# EUROPEAN PATENT APPLICATION

(11) **EP 3 566 718 A1**
(43) Date of publication of application: **13.11.2019**
(21) Application number: 18170999.9
(22) Date of filing: 07.05.2018
(51) Int. Cl.: A61K 39/395, A61K 39/39, C07K 16/28, C12N 15/117, A61P 35/00, A61K 39/00

(54) **A PHARMAZEUTICAL COMBINATION (TREG DEPLETING AGENT, CHECKPOINT INHIBITOR, TLR9 AGONIST) FOR USE IN THE TREATMENT OF CANCER**

(71) Applicant: Universitätsmedizin der Johannes Gutenberg-Universität Mainz, 55131 Mainz (DE)
(72) Inventor: ROSIGKEIT, Sebastian, 55131 Mainz (DE); BOCKAMP, Ernst-Otto, 55126 Mainz (DE); SCHUPPAN, Detlef, 55122 Mainz (DE)
(74) Representative: Patentanwälte Dr. Keller, Schwertfeger Partnerschaft mbB

(57) **Abstract**

The present disclosure relates to a pharmaceutical combination, comprising the components: a) at least one regulatory T cell (Treg) -depleting agent, b) at least one Toll-like receptor 9 (TLR9) agonist, c) one or more immune checkpoint inhibitors, for use in the treatment of cancer in humans or non-human mammals.

Sequence Listing:

<110> Universitätsmedizin der Johannes Gutenberg-Universität
Mainz

<120> Pharmaceutical Combination for use in the treatment of cancer

<130> Therapie X

<160> 12

<170> PatentIn version 3.5

<210> 1
<211> 24
<212> DNA
<213> Homo sapiens

<400> 1
tcgtcgtttt gtcgttttgt cgtt 24

<210> 2
<211> 20
<212> DNA
<213> Homo sapiens

<400> 2
gggggacgat cgtcgggggg 20

<210> 3
<211> 22
<212> DNA
<213> Homo sapiens

<400>  3
tcgtcgtttt cggcgcgcgc cg                                                22

<210>  4
<211>  20
<212>  DNA
<213>  Mus musculus

<400>  4
ggggtcaacg ttgagggggg                                                   20

<210>  5
<211>  20
<212>  DNA
<213>  Mus musculus

<400>  5
tccatgacgt tcctgacgtt                                                   20

<210>  6
<211>  20
<212>  DNA
<213>  Mus musculus

<400>  6
tccatgacgt tcctgatgct                                                   20

<210>  7
<211>  23
<212>  DNA
<213>  Homo sapiens

<400> 7
tcgacgttcg tcgttcgtcg ttc 23

<210> 8
<211> 22
<212> DNA
<213> Bos taurus

<400> 8
tcgtcgttgt cgttttgtcg tt 22

<210> 9
<211> 26
<212> DNA
<213> Homo sapiens

<400> 9
tcgcgacgtt cgcccgacgt tcggta 26

<210> 10
<211> 21
<212> DNA
<213> Homo sapiens

<400> 10
ggggacgacg tcgtgggggg g 21

<210> 11
<211> 25
<212> DNA
<213> Homo sapiens

<400> 11

tcgtcgtcgt tcgaacgacg ttgat 25

<210> 12
<211> 29
<212> DNA
<213> Homo sapiens

<400> 12
tcgcgaacgt tcgccgcgtt cgaacgcgg 29

SEQUENCE LISTING

<110>  Universitätsmedizin der Johannes Gutenberg-Universität
       Mainz

<120>  Pharmaceutical Combination for use in the treatment of cancer

<130>  Therapie X

<160>  12

<170>  PatentIn version 3.5

<210>  1
<211>  24
<212>  DNA
<213>  Homo sapiens

<400>  1
tcgtcgtttt gtcgttttgt cgtt                                              24


<210>  2
<211>  20
<212>  DNA
<213>  Homo sapiens

<400>  2
gggggacgat cgtcgggggg                                                   20


<210>  3
<211>  22
<212>  DNA
<213>  Homo sapiens

<400>  3
tcgtcgtttt cggcgcgcgc cg                                                22


<210>  4
<211>  20
<212>  DNA
<213>  Mus musculus

<400>  4
ggggtcaacg ttgagggggg                                                   20


<210>  5
<211>  20
<212>  DNA
<213>  Mus musculus

<400>  5
tccatgacgt tcctgacgtt                                                   20


<210>  6
<211>  20
<212>  DNA
<213>  Mus musculus

<400>  6
tccatgacgt tcctgatgct                                                    20


<210>  7
<211>  23
<212>  DNA
<213>  Homo sapiens

<400>  7
tcgacgttcg tcgttcgtcg ttc                                                23


<210>  8
<211>  22
<212>  DNA
<213>  Bos taurus

<400>  8
tcgtcgttgt cgttttgtcg tt                                                 22


<210>  9
<211>  26
<212>  DNA
<213>  Homo sapiens

<400>  9
tcgcgacgtt cgcccgacgt tcggta                                             26


<210>  10
<211>  21
<212>  DNA
<213>  Homo sapiens

<400>  10
ggggacgacg tcgtgggggg g                                                  21


<210>  11
<211>  25
<212>  DNA
<213>  Homo sapiens

<400>  11
tcgtcgtcgt tcgaacgacg ttgat                                              25


<210>  12
<211>  29
<212>  DNA
<213>  Homo sapiens

<400>  12
tcgcgaacgt tcgccgcgtt cgaacgcgg                                          29

## Description

The present invention relates to a pharmaceutical combination and a kit of parts for use in the treatment of cancer in humans or non-human mammals.

Most cancers have an overall dismal prognosis and only when diagnosed early, surgery and ablative therapies may offer a cure (Siegel et al., 2017). In many cases, however, and in particular with regard to lung cancer, a malignant disease is only diagnosed at an advanced stage, when chemotherapy, radiation and in some cases tyrosine kinase inhibitors provide palliation and prolong survival (Ettinger et al., 2016; Novello et al., 2016). Immune vaccination with immune checkpoint inhibitor combinations remains the only available alternative treatment option (Antonia et al., 2017; Brahmer et al., 2015; Carbone et al., 2017; Govindan et al., 2017; Hellmann et al., 2017).

Under physiological conditions, both stimulatory and inhibitory pathways regulate the inflammatory immune response to pathogens and maintain tolerance to self-antigens. These are regulated by a diverse set of immune checkpoints, thereby protecting healthy tissues from damage. These checkpoints can be co-opted by malignant tumours to dampen the immune response and evade destruction by the immune system. Typical immune checkpoint inhibitors that show clinical progress are cytotoxic T-lymphocyte antigen 4 (CTLA-4) and programmed cell death-1 (PD-1), which have been the initial focus of anticancer agent development agent. PD-1 and co-inhibitory receptors such as CTLA-4, B and T lymphocyte attenuator (BTLA; CD272), T cell Immunoglobulin and mucin domain-3 (TIM-3), lymphocyte activation gene-3 (LAG-3; CD223), and others are often referred to as a checkpoint regulators.

The CTLA-4 and PD-1 pathways operate at different stages of the immune response. In contrast to the effect of CTLA-4 on early T-cell activation, the PD-1 pathway appears to impact the T-cell response at the (later) effector stage. PD-1 is upregulated on T cells after persistent antigen exposure, typically in response to chronic infections or tumours. PD-L1 and PD-L2, the ligands for PD-1, can be expressed by tumour cells, as well as several other hematopoietic and non-hematopoietic cell types.

Clinical inhibition of CTLA4 has been performed with ipilimumab and tremelimumab. However, in aggregating data for patients treated with ipilimumab, it appears that there may be a plateau in survival at approximately 3 years. Thereafter, patients who remain alive at three years may experience a persistent long-term survival benefit, including some patients who have been followed for up to 10 years. One major drawback to such an anti-CTLA-4 mAb therapy are possible autoimmune toxicities due to an over-exuberant immune system which has lost the ability to turn itself down. It has been reported that up to 25% of patients treated with ipilimumab developed serious grade 3-4 adverse events/autoimmune-type side effects including dermatitis, enterocolitis, hepatitis, endocrinopathies (including hypophysitis, thyroiditis, and adrenalitis), arthritis, uveitis, nephritis, and aseptic meningitis. Accordingly, while checkpoint inhibitors are extremely effective at treating cancers in the responding subject population, approximately 75% of cancer subjects will not respond to the therapy. In addition, even in the responding population the response is not always complete or optimal.

A clinical study reported by Herbst et al. (Herbst et al., 2014) was designed to evaluate the single-agent safety, activity and associated biomarkers of PD-L1 inhibition using a humanized monoclonal anti-PD-L1 antibody. In contrast to the anti-CTLA-4 experience, anti-PD-1 therapy appears to be better-tolerated and induces a relatively lower rate of autoimmune-type side effects.

Thus, clinical application of these first generation anti-cancer immune therapeutics demonstrated that only a fraction of patients (for example in lung cancer about 20%) respond to immune checkpoint blocking therapies (Brahmer et al., 2015; Brahmer et al., 2012; Topalian et al., 2012).

The failure of classical treatment strategies and the limited responsiveness to first generation checkpoint blocking immune therapies highlight that novel and better treatment options are required for cancer therapy. Given the fact that the responsiveness and clinical success of immune checkpoint regulators alone is limited, synergistic therapies, i.e. combining immune checkpoint regulators with additional immune-regulatory pathways, represent a reasonable approach to increase the efficacy of anti-cancer immune therapies. Since natural immune responses are based on both adaptive and innate immunity, anti-cancer immune vaccines benefit from the combinatorial action of therapeutic agents stimulating both adaptive (T and B cells) as well as innate (dendritic cells, innate lymphoid cells, macrophages, NK cells and granulocytes) immune responses.

One group of synergistic co-therapeutics capable of reshaping the tumour microenvironment and enhancing maturation, antigen presentation and priming of tumour-specific cytotoxic lymphocytes are Toll-like receptors (TLRs). TLRs are present on many cells of the immune system and have been shown to be involved in the innate immune response (Hornung et al., 2002). TLRs are a key means by which vertebrates recognize and mount an immune response to foreign molecules and also able to link the innate and adaptive immune responses (Akira et al., 2001; Medzhitov, 2001). Some TLRs are located on the cell surface to detect and initiate a response to extracellular pathogens and other TLRs that are located inside the cell to detect and initiate a response to intracellular pathogens. In addition, TLRs sense danger signals and can promote immune-stimulatory anti-cancer responses (Li et al., 2017).

TLR agonists have the capability to bridge innate and adoptive anti-cancer immune responses. However, increasing evidence suggests that different TLRs may have different and, in some cases, unwanted side effects on cancer development (Shi et al., 2016). For this reason, the application of TLRs as immune-enhancing agents in combination therapies must be elucidated.

Among TLRs, TLR9 appears to be a good candidate to enhance immune therapies because TLR9 stimulation promotes an innate immune response that is characterized by the production of Th1 and pro-inflammatory cytokines (Scheiermann and Klinman, 2014). TLR9 is known to recognize unmethylated deoxycytidylate-phosphate-deoxyguanylate (CpG) motifs commonly found in bacterial and viral DNA, synthetic IMO-2055 (EMD1201081) and in immune modulatory oligonucleotides (such as IMO-2125 or IMO-20155) and other synthetic oligonucleotides that mimic unmethylated CpG sequences in bacterial or viral DNA (Gosu et al., 2012; Hemmi et al., 2000). In addition, TLR9 agonists that contain a phosphodiester backbone and fold in a dumbbell-like structure known as double stem-loop immunomodulators (dSLIMs) such as MGN-1703 and MGN-1706 are acting as TLR9 agonists (Gosu et al., 2012; Mikulandra et al., 2017). Moreover, naturally occurring agonists of TLR9 have been shown to produce anti-tumour activity (e.g. tumour growth and angiogenesis) resulting in an effective anti-cancer response, e.g. anti-leukemia (Smith, J. B. and Wickstrom, E. (1998) J. Natl. Cancer Inst. 90:1146-1154) (Smith and Wickstrom, 1998). TLR9 targeting is especially convenient for therapeutic lung applications since TLR9 is expressed in bronchial epithelium, vascular endothelium, alveolar septal cells, alveolar macrophages, dendritic cells and B cells in mice and men (Scheiermann and Klinman, 2014; Schneberger et al., 2013). In addition, TLR9 agonists have been used in clinical trials. Published data shows no adverse toxic effects and in some cases also small clinical benefits (Carpentier et al., 2010; Karbach et al., 2011; Ursu et al., 2017).

In view of the drawback of individual TLR9 and checkpoint inhibitor therapies, combined therapies have been developed in which immune checkpoint modulators or checkpoint inhibitors are co-administered to cancer patients together with one or more TLR9 agonists.

WO 2016 146 261 A1 describes a combination of an immune checkpoint modulator and a complex comprising a cell penetrating peptide, at least one antigen or antigenic epitope, and a TLR peptide agonist for use in medicine, in particular in the prevention and/or treatment of cancer. Moreover, the present invention also provides compositions, such as a pharmaceutical compositions and vaccines, which are useful in medicine, for example in the prevention and/or treatment of cancer.

EP 3 204 04 0A1 describes methods of inducing an immune response to cancer comprising co-administering to a cancer patient one or more TLR9 agonists and one or more checkpoint inhibitors.

WO 2015 069 770 A1 describes a method of treating cancer or initiating, enhancing, or prolonging an antitumour response in a subject in need thereof comprising administering to the subject a therapeutic agent in combination with an agent that is a checkpoint inhibitor.

An additional obstacle that prevents successful cancer immune therapies using checkpoint inhibitors and/or TLRs are immune-suppressive regulatory T cells (Tregs or Treg cells) (Tanaka and Sakaguchi, 2017). The natural function of Tregs is to inhibit pathological autoimmunity. In cancer, the immune system is re-programmed in order to generate Treg cells that serve to protect the tumour against immunological attack. Immune responses result in an upregulation of co-inhibitory receptors to suppress an immune response. Typical examples of upregulated co-inhibitory molecules on T-cells are CTLA-4, PD-1, TIM-3 and LAG-3. The finding of co-inhibitory receptors has led to the development of antibodies against these receptors in order to allow for T-cell activation to continue and not to be inhibited by Treg cells. By this mechanism, T-cells start attacking various targets.

By now, there is no reliable therapy or combination of active agent that would result in an effective therapy of cancer using TLR or checkpoint inhibitors avoiding the drawback mentioned above.

It is therefore an object of the present invention to provide a therapeutic combination of pharmaceutically active components to enhance the efficiency of anti-tumour vaccination and to improve anti-cancer immune responses.

This object is solved by a pharmaceutical combination comprising the individual components as defined in claim 1. Preferred embodiments of the pharmaceutical combination are part of the sub-claims.

The pharmaceutical combination of the present invention comprises the three following components:
a) at least one regulatory T cell (Treg)-depleting agent,
b) at least one Toll-like receptor 9 (TLR9) agonist,
c) one or more immune checkpoint inhibitors.

Each component is preferably separated from the other component and administered independently from each other to a human or non-human mammalian subject. As part of an alternative embodiment, component b) and component c) may also be combined together to a subject suffering from cancer in a single administration dose in order to achieve a therapeutic effect upon administration.

In view of the inhibitory therapeutic effect on the growth and progression of tumour cells, the present invention specifically relates to an inventive pharmaceutical combination for use in the treatment of cancer in humans or non-human mammals. Essentially, the pharmaceutical combination is suitable for any vertebrate, which has similar signalling and checkpoint pathways as present in humans and other mammals. The pharmaceutic combination of the present invention is preferably designed in form of a kit of parts in which the at least one Treg-depleting agent, the at least one TLR-9 agonist and the one or more immune checkpoint inhibitors are contained in different compartments, containers or injection solutions applicable to a subject suffering from cancer.

The Treg-depleting agent according to component a) will either remove or inactivate regulatory T cells (Tregs). This measure will precondition the tumour microenvironment and enhance the efficiency of anti-tumour vaccination. Preferably, the Treg-depleting agent of component a) is a surface antigen-depleting antibody or antibody fragment, for example an Fc-fragment that comprises the variable heavy and light chains domains (V_{L} and V_{H}) required for antigen recognition and binding. In a preferred embodiment, the Treg-depleting agent of component a) is an antibody or antibody fragment directed against CD25, CD15s, GITR, CCR4, CTLA-4, OX-40, LAG3, GARP, ZAP-70 or PD-1 surface antigen. Depleting antibodies or antibody fragments directed to surface antigens are specific to Tregs and have the capability to deplete regulatory T cells to improve anti-cancer immune responses. For example, the Treg-depleting antibody αCCR4 is able to deplete effector Tregs without showing adverse toxic side effects in humans.

As an alternative or in addition to the Treg-depleting target structures, also an inhibition of phosphatidylinositol-4,5-bisphosphate 3-kinase delta (PI3Kδ) allows to specifically target Treg cells, resulting in an enhanced immunotherapeutic effect in mammals. The inventive combination of the application of a Treg-depleting agent followed by an application of TLR9 agonists and checkpoint inhibitors is a promising approach for the treatment of cancer in humans and non-human mammals as it will significantly enhance the effectiveness of the therapy and the survival of the treated patients. As such, the inventive synergistic therapy combines the action of immune checkpoint regulating inhibitors such as PD-1 and/or CTLA4 together with additional immune-regulatory pathways. This approach has the potential to improve the therapeutic action of anti-cancer immune vaccinations by specifically targeting or depleting Tregs, thereby enhancing immune responsiveness as a result of TLR stimulation. The present invention is supported by preclinical data illustrating that a prior depletion of Tregs or effector Treg cells will stop tumour progression following injections of immune checkpoint regulating agents and TLR9 agonists. For this purpose, a mouse model has been generated to evaluate the synergistic effects of a prior depletion of Tregs when applying a combination of immune checkpoint inhibitors and TLR9 agonists. A depletion of Tregs prior to the subsequent injection of checkpoint inhibiting antibodies and TLR9 agonists in mice reduces the tumour burden already after one month of treatment.

Most significantly, a Treg depletion by Treg-depleting agent alone or the application of TLR9 agonists and/or checkpoint inhibitors without prior Treg cell-depletion or a single injection of checkpoint inhibitors alone did not produce the drastic tumour reduction as seen with the pharmaceutical combination and the mode of administration according to the present invention. The therapeutic effect of the application of a Treg cell-depletion agent subsequent to one or more TLR9 and immune checkpoint inhibitor injections is synergistic, thus resulting in a manifest tumour reduction. As the general mode of action of the components of the pharmaceutical combination of the invention is similar in mice and men, the data presented herein are not only applicable to human patients, but to mammals in general. Furthermore, as the mode of action of all components of the pharmaceutical combination of the present invention is antigen-independent and not relying of the expression of specific tumour neo-antigens that are specifically targeted by an antigen-specific vaccination, the combination of the invention will not only work in regard to lung cancer, but is suitable in the treatment of any kind of malignant cancer. Preferred forms of cancer to be treated are lung cancer, melanoma, renal cancer, hepatocellular carcinoma and other cancer types.

The second component b) of the inventive pharmaceutical combination can be any of the known TLR9 agonists. In a preferred embodiment, the TLR9 agonist of component b) is composed of CpG oligodeoxynucleotides (CpG ODN) containing one or more unmethylated CpG dinucleotides. CpG ODNs are synthetic oligonucleotides that contain unmethylated CpG ODNs in particular sequence contexts (CpG motives). These CpG motives are present at an approximately at 20-fold greater frequency in bacterial DNA compared to mammalian DNA. CpG ODNs are recognised by TLR9 leading to strong immuno-stimulatory effects.

Preferably, the TLR9 agonist as used in the pharmaceutical combination comprises CpG ODN which is composed of the formula CCx(not-C)(not-C)xxGGG, wherein x is any base selected from the group consisting of modified or unmodified A, T, C, G or derivatives thereof. In an embodiment, the CpG ODN of the invention contains one or more nuclease-resistant phosphorothioate oligodeoxynucleotides.

Preferably, the CpG ODN utilized in the inventive combination as TLR9 agonist comprises one or more of the following nucleic acid sequences:
5'-TCGTCGTTTTGTCGTTTTGTCGTT-3' (SEQ ID NO: 1),
5'-GGGGGACGATCGTCGGGGGG -3' (SEQ ID NO: 2),
5'-TCGTCGTTTTCGGCGCGCGCCG-3' (SEQ ID NO: 3),
5'-GGGGTCAACGTTGAGGGGGG -3' (SEQ ID NO: 4),
5'-TCCATGACGTTCCTGACGTT-3' (SEQ ID NO: 5),
5'-TCCATGACGTTCCTGATGCT-3' (SEQ ID NO: 6),
5'-TCGACGTTCGTCGTTCGTCGTTC-3' (SEQ ID NO: 7),
5'-TCGTCGTTGTCGTTTTGTCGTT-3' (SEQ ID NO: 8),
5'-TCGCGACGTTCGCCCGACGTTCGGTA-3 (SEQ ID NO: 9),
5'-GGGGACGACGTCGTGGGGGGG-3' (SEQ ID NO: 10),
5'-TCGTCGTCGTTCGAACGACGTTGAT-3' (SEQ ID NO: 11),
5'-TCGCGAACGTTCGCCGCGTTCGAACGCGG-3' (SEQ ID NO: 12).

The use of human TLR9, mouse TLR9 or other mammalian TLR9 falling in anyone of the group of known class A, B or C agonists is preferred.

In an alternative embodiment, the TLR9 agonists of component b) can also be a single-stranded or double-stranded genomic DNA isolated from *Escherichia coli* or other prokaryotes and viruses with the ability to bind to TLR9 or those that mimic unmethylated CpG sequences in bacterial or viral DNA TLR9 agonists such as phosphodiester backbone and fold in a dumbbell-like structures known as double stem-loop immunomodulators (dSLIMs) and are acting as TLR9 agonists.

According to the present invention, the TLR9 agonist or a combination of different TLR9 agonists can be given in a single injection dose or in repeatedly administered injection doses either alone or in combination with one or more checkpoint inhibitors. In a variant, the TLR9 agonists are therefore provided in a separate injection solution without the presence of any immune checkpoint inhibitor. In an alternative embodiment, the TLR9 agonists and the checkpoint inhibitor are provided together in a single injection solution as part of the pharmaceutical combination of the present invention. In this composition, more than one checkpoint inhibitor is combined with another checkpoint inhibitor and/or TLR9 agonist in order to maximise the possible therapeutic effects. The exact composition of the pharmaceutical combination or its mode of administration, however, will depend on the kind of cancer to be treated. As illustrated herein, the therapeutic effects and the suppression of adverse side effects strongly depend on the prior depletion of regulatory T cells. If depletion of Treg cells reaches its maximum, the application of TLR9 and/or checkpoint inhibitors and their therapeutic effects will be most efficient.

Suitable checkpoint inhibitors as utilised in the pharmaceutical combination of the present invention preferably inhibit a checkpoint protein selected from the group consisting of CTLA4, PD-1, PD-L1, PD-L2, LAG3, B7-H3, B7-H4, KIR, OX40, IgG, IDO-1, IDO-2, CEACAM1, TNFRSF4, OX40L, TIM3, BTLA, HVEM, TIM3, GAL9, LAG3, VISTA, KIR, 2B4, CD 160, CGEN-15049, CHK 1, CHK2, A2aR, and B-7 or combinations thereof. In a preferred embodiment, the immune checkpoint inhibitor is a combination of αCTLA-4 and αPD-1 antibodies, preferably diluted in a single injection solution. In a preferred mode of administration, αCTLA-4 and αPD-1 antibodies are diluted 1:1 in the single injection solution.

In an alternative embodiment, the Treg-depleting agent can also be injected during or subsequent to the application of TLR9 agonists and/or immune checkpoint inhibitors. For example, it is possible to specifically inhibit Tregs using PI3Kδ inhibitors in order to further increase the success of the therapy, which can be measured by a reduction of tumour progression. As such, also the continuous provision of Tregs can be controlled, e.g. if the patient shows an excessive immune reaction upon application of the checkpoint inhibitor.

Any mode of administration can be part of the inventive dosage regimen which may be part of the inventive combination, which preferably is designed as a kit of parts. Each component of the kits of parts acts in combination with the others in order to achieve the desired therapeutic effect, i.e. to reduce tumour progression in affected tumour tissue.

In a preferred embodiment, the combination of the invention or the kit of parts further comprises a dosage regime which provides that component a) is to be administered once or repeatedly before component b) and component c), and wherein optionally component b) is to be administered once or repeatedly before administering component c) to a cancer patient, such as a human patient or animal patient. In a preferred embodiment, the dosage regimen provides that
i. component a) is to be injected 5 to 9 days prior to day +1,
ii. component b) is to be injected every 3^{rd} or 4^{th} day starting at day +1 by one or more injections,
iii. component c) is to be injected every 3^{rd} or 4^{th} day starting at day +1 by one or more injections.

Usually Treg cell-depletion reaches its strongest effect seven to ten days after the last injection. It is preferred that the checkpoint inhibitor application is repeated for a total of more than 2 injections, preferably for a total of 5 injections starting at day +1. The application of TLR9 agonists occurs every third or fourth day starting at day +1.

The provision of the pharmaceutical combination in form of a kit of parts is preferred. In such a kit of parts, each component is provided in a separate compartment or injection solution. Preferably, all three components a) to c) are enclosed in separate, independent injection solutions for subsequent injections, preferably repeated injections, to a cancer patient.

The present invention also relates to an *ex-vivo* method for diminishing growth of tumour cells in a tissue, comprising the steps of:
i. application of at least one regulatory T cell (Treg)-depleting agent,
ii. application of at least one Toll-like receptor 9 (TLR9) agonist,
iii. application of one or more immune checkpoint inhibitors, wherein the at least one TLR9 agonist is subsequently applied after Treg cell depletion, and wherein the one or more immune check point inhibitors are applied subsequently to or simultaneously with the at least one TLR9 agonist.

The application of the Treg-depleting agent can be done in the course of a preconditioning of the micro-environment using surface-specific antibodies or antibody fragments, or by the application of phosphatidylinositol-4,5-bisphosphate 3-kinase delta (PI3Kδ) inhibitors prior to or during TLR9 and/or checkpoint inhibitor application.

The present invention will be further illustrated by the following examples.

### Examples:

As a proof of concept, a pharmaceutical combination consisting of anti-CD25 antibodies, TLR9 agonists and checkpoint inhibitors were used as components to achieve the desired therapeutic synergy in a mouse model, which results can also be applied to other mammals, including humans.

Anti-CD25 antibodies were used as a preconditioning measure to deplete immune-suppressive Tregs. TLR9 agonists were used to stimulate the innate arm of the immune system. A combination of the known CTLA-4 and PD-1 checkpoint inhibitors were used to boost T cell activity and to reduce the presence or activity of regulatory T cells. αCD25 injection solution (anti-mouse CD25) was diluted in PBS to a concentration of 500 µg/200µl as the initial step of depletion of Treg. After Treg depletion, several injections of immune checkpoint regulating agents together with TLR9 agonists were applied. The therapeutic effect was measured using a preclinical mouse model (SCKP mouse model), which contains a conditional gene switch that sets off oncogenic K-RasG12V and inactivates the p53 tumour suppressor gene in lung epithelial cells upon TAM injections (Cre/loxP system). This setting recapitulates the most common genetic driver mutations found in human lung cancer. In the Scgb1a1-CreER^{T2}/K-Ras^{LSLG12V}/p53^{fl/fl}. (SCKP) mouse model, lung cancer was induced by a single injection of Tamoxifen, which activates oncogenic K-RasG12V and inactivates p53 tumour suppression function in lung epithelial Clara cells and broncho-alveolar stem cells (BASCS). Therapy started at four months following Tamoxifen injection when about 20 % of the lung consisted of growing tumours. One month after therapy initiation (i.e. 5 months after Tamoxifen injection), the inventors quantified the therapeutic effect of this dose regime.

When therapeutic treatment was started, the lung tumour burden was > 20% of the total lung mass (untreated 4 months; 4M). After one month (5M), mice receiving no therapy had a mean lung tumour burden of about 30% (untreated 5 months). The tumour burden was markedly reduced on average to about 10% of the total lung mass when therapeutic treatment (αCD25 preconditioning followed by CpG/checkpoint treatment) was applied and after one month of treatment the tumour mass reduction determined. Also no obvious adverse toxicities and side effects were noted in mice undergoing therapeutic treatment.

The preclinical data are further exemplified in the following Figures:

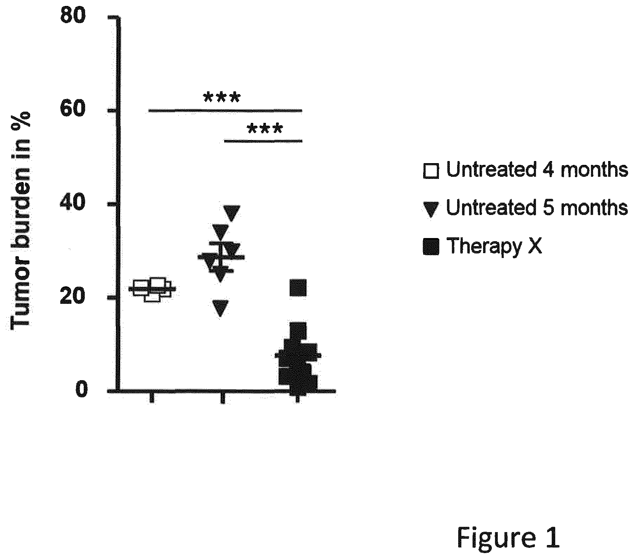

Figure 1 shows the reduction of tumour burden after one month of treatment using the pharmaceutical combination of the present invention. The graph indicates the mean tumour burden in mice when treatment was started (white boxes), tumour progression without treatment one month later (inverted black triangles) and one month after application of the inventive combination (black boxes). Each symbol indicates one individual mouse. ***, p ≤ 0.001.

As a control measure, also the effect of Treg depletion alone or the application of TLR9 agonists and checkpoint inhibitors without prior Treg depletion has been tested.

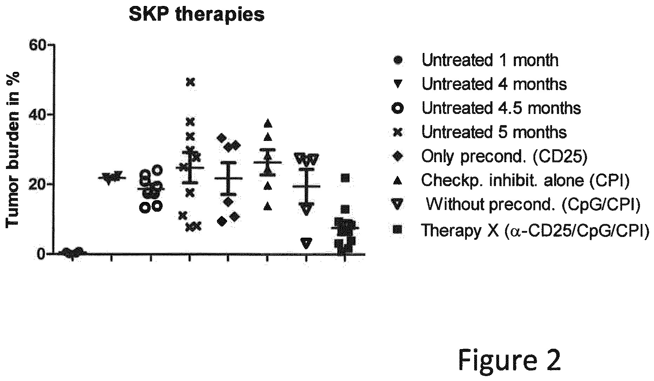

Figure 2 shows the therapeutic effects of the inventive combination of preconditioning alone (anti-CD25), the action of TLR9 and checkpoint inhibitors without prior preconditioning, the effect of checkpoint inhibitors alone (antibodies against PD-1 and CTLA-4) and the tumour reduction as a result of the inventive combination that comprises the application of a Treg-depleting agent followed by TLR9 agonists and checkpoint inhibitor administration. The therapeutic effect of the inventive combination is synergistic. Tumour reduction is only reached when Treg-depletion is combined with TLR9 agonists and checkpoint inhibitors.

The graph of Fig. 2 indicates the mean tumour burden in mice one month after inducing the K-Ras/p53 gene switch (black dots), when treatment was started (black inverted triangles, four months after TAM injection), 15 days (white rings) and one month (black crosses) later without treatment. Results depicting treated groups are: Black diamonds showing the therapeutic action of preconditioning alone (anti-CD25), black triangles indicating the action of TLR9 and checkpoint inhibitors without preconditioning, inverted white triangles representing the effect of checkpoints alone (antibodies against PD-1 and CTLA-4) and black boxes showing tumour reduction as a result of the inventive combination. Each symbol indicates one individual mouse.

The data clearly show that Treg-depletion (preconditioning by anti-CD25 Treg-depletion) alone or the application of TRL9 agonists (CpG oligodeoxynucleotides) and checkpoint inhibitors (PD-1 and CTLA-4) without Treg-depletion or the administration of checkpoint inhibitors alone did not produce the same drastic tumour reduction as seen upon treatment with the combination of the present invention.

### Material and methods:

### Mice

For conditional expression of oncogenic K-RasG12V and conditional inactivation of P53 gene function in lung epithelial cells, the inventors intercrossed the LSL-K-RasG12V knock-in mouse model (Guerra et al., 2003), the conditional p53 knock-in mouse model (Jonkers et al., 2001) and the Scgb1a1-CreER^{T2} driver knock-in mouse model that contains the CreERT2 Cre-recombinase fusion gene (Hameyer et al., 2007) under the transcriptional control of the Secretoglobin 1a1 gene locus (Rawlins et al., 2009). The conditional LSL-K-RasG12V mouse genotype used for testing the inventive pharmaceutical combination and dosage regimen is identical to the one described by Guerra and colleagues but contains an additional knock-in in the ROSA26 gene locus, allowing the conditional activation of the EYFP reporter upon TAM injection. All conditional genes (oncogenic K-RasG12V, loss of p53 function and the two co-reporter genes EYFP and lacZ) are activated upon intraperitoneal (i.p.) injection of Tamoxifen (TAM). Mice combining all three genotypes (the Scgb1a1-CreER^{T2}/LSL-K-RasG12V/p53^{fl/fl} model) were on a C57BL/6 Jackson genetic background.

***Additional information:*** TAM injection in Scgb1a1-CreER^{T2}/LSL-K-RasG12V/p53^{fl/fl} mice promotes the onset of lung cancer and the progressive onset of lung tumourigenesis. Using defined amounts of TAM results in complete penetration of the lung cancer phenotype in all mice. Both the kinetics and the induced phenotype are highly reproducible between individual mice and depend on the amount of TAM injected.

### Induction of Cre-recombinase activity in mice using tamoxifen (TAM)

For preparing TAM stock solution, 100 ml of sunflower oil was autoclaved. First, 1 g of TAM powder was diluted in 10 ml ethanol at 37 °C. This dilution was mixed with 90 ml of autoclaved sunflower oil at room temperature and the mixture was shaken overnight. The homogenous solution was aliquoted in Eppendorf tubes and stored at -20 °C. For treatment of adult mice (8-12 weeks old), 100 µl of TAM solution were administered as a single i.p. injection per mouse. For short-term usage, TAM was stored at 4 °C up to 5 days. TAM was protected from light exposure at all times.

### Whole mount LacZ staining in transparent lungs to visualize tumour burden and tumour load quantification

Lungs from mice were dissected five months after a single TAM injection and analysed one month after therapy start, washed in ice cold PBS (pH 7.4) and fixed in acetone for 8 h at 4 °C. Next, lungs were washed twice for 5 min in PBS and left for 8 to 12 h in X-gal buffer solution (pH 7.4 without X-gal) on a rocking platform at 4°C. This was necessary to completely infiltrate the tissue and to establish a pH of 7.4. Lungs were transferred into 20 ml of X-gal staining solution and incubated 6 to 12 h shaking at 100 rpm at 37 °C and in the dark. In a next step, lungs were washed once in PBS for 5 min and then fixed over-night until one week at 4 °C in a fixation buffer containing 4% formaldehyde and 1% glutaraldehyde. For dehydration lungs were first washed three times in PBS for 5 min using a rocking platform at room temperature and transferred into different methanol/PBS buffers using 50 ml volume for each lung (at RT). Subsequently, lungs were placed into 25 % methanol in PBS for one hour or until the organ was sinking to the bottom of the glass bottle. This step was repeated with 50% and 75% of methanol in PBS and three times with a concentration of 100% methanol. Ultimately, lungs were incubated for at least 12 h in 100% methanol at 4°C to ensure water free tissues. For rendering the pre-treated lungs transparent, lungs were incubated in 20 ml of a 2:1 mixture of benzyl benzoate and benzyl alcohol for a few minutes. Images of transparent lungs were immediately taken using a binocular equipped with a strong light source (3200 K light and 60 ms time of light exposure) and recorded at a magnification of 1.6 x 10. The ImageJ software package from the NIH was used to quantify the tumour load (https://imagej.net/Welcome) using images of transparent lungs. In all cases, Scgb1a1-CreER^{T2}/LSL-K-RasG12V/p53^{fl/fl} mice were induced with one single i.p. injection of 100 µl TAM to set off lung tumourigenesis and were treated with the inventive pharmaceutical combination and dosage regimen four months later. Analysis was in all cases at five months after TAM injection.

### Composition of the pharmaceutical combination

To achieve therapeutic synergy, the inventive combination was composed of anti-CD25 antibodies (to deplete immune-suppressive regulatory T cells), TLR9 agonists (to stimulate the innate arm of the immune system) and checkpoint inhibitors (to boost T cell activity and to reduce regulatory T cells).

### Depletion of regulatory T cells

For depletion of regulatory T cells in mice, 500 µg of InVivoMAb anti-mouse CD25 (BioXCell) was injected on two consecutive days i.p.) at day -9 and -8 (at day +1 TLR9 agonist and PC-1/CTLA-4 antibodies are injected for the first time). Depletion reaches its strongest effect 9 days after the last injection as described (Onizuka et al., 1999). Alternatively (but not used in our initial experimental setup), regulatory T cells can be depleted and immune function boosted using phosphatidylinositol-4,5-bisphosphate 3-kinase delta (PI3Kδ) inhibitors (Ahmad et al., 2017; Bowers et al., 2017).

### TLR-9 agonist application

For systemic stimulation of TLR-9 in mice, CpG islands were produced and phosphorothioate-stabilized at Metabion International AG. The sequence 5'-TCCATGACGTTCCTGATGCT-3' was used since this sequence was shown to efficiently reverse regulatory T cell-mediated CD8 tolerance in mice (Yang et al., 2004). During immunotherapeutic treatment, 50 µg of CpGs were injected into experimental animals every 3^{rd} or 4^{th} day starting at day +1.

### Checkpoint mediator application

To enhance effective T cell responses, 100 µg of αCTLA-4 (Clone: 9D9, Catalog: BE0164) in combination with 100 µg of αPD1 (Clone RMP1-14, Catalog: BE0146) were injected every 3^{rd} or 4^{th} day into experimental animals during immunotherapeutic treatment for a total 5 injections starting at day +1.

### Stock solutions:

*aCD25 injection solution:* InVivoMAb anti-mouse CD25 (IL-2Rα) (Clone: PC-61.5.3, Catalog: BE0012) was purchased from BioXCell and diluted in PBS to a concentration of 500 µg/200 µl.
*CpG injection solution:* CpGs were produced and lyophilized at Metabion International AG and diluted with autoclaved H₂O_{dd} to a concentration of 50 µg/200 µl.
*αCTLA-4 stock solution:* InVivoMAb anti-mouse CTLA-4 (Clone: 9D9, Catalog: BE0164) was purchased from BioXCell and diluted in PBS to a concentration of 100 µg/50 µl.
*αPD1 stock solution:* InVivoMAb anti-mouse PD-1 (Clone RMP1-14, Catalog: BE0146) was purchased from BioXCell and diluted in PBS to a concentration of 100 µg/50 µl. *Checkpoint mediator injection solution:* CTLA-4 and PD1 stock solution were diluted 1:1 prioi to injection into experimental animals (100 µl total injection volume).
Ahmad, S., Abu-Eid, R., Shrimali, R., Webb, M., Verma, V., Doroodchi, A., Berrong, Z., Samara, R., Rodriguez, P. C., Mkrtichyan, M., and Khleif, S. N. (2017). Differential PI3Kδ Signaling in CD4. Cancer Res 77, 1892-1904.
Akira, S., Takeda, K., and Kaisho, T. (2001). Toll-like receptors: critical proteins linking innate and acquired immunity. Nat Immunol 2, 675-680.
Antonia, S. J., Villegas, A., Daniel, D., Vicente, D., Murakami, S., Hui, R., Yokoi, T., Chiappori, A., Lee, K. H., de Wit, M., et al. (2017). Durvalumab after Chemoradiotherapy in Stage III Non-Small-Cell Lung Cancer. N Engl J Med 377, 1919-1929.
Bowers, J. S., Majchrzak, K., Nelson, M. H., Aksoy, B. A., Wyatt, M. M., Smith, A. S., Bailey, S. R., Neal, L. R., Hammerbacher, J. E., and Paulos, C. M. (2017). PI3Kδ Inhibition Enhances the Antitumor Fitness of Adoptively Transferred CD8. Front Immunol 8, 1221.
Brahmer, J., Reckamp, K. L., Baas, P., Crinò, L., Eberhardt, W. E., Poddubskaya, E., Antonia, S., Pluzanski, A., Vokes, E. E., Holgado, E., et al. (2015). Nivolumab versus Docetaxel in Advanced Squamous-Cell Non-Small-Cell Lung Cancer. N Engl J Med 373, 123-135.
Brahmer, J. R., Tykodi, S. S., Chow, L. Q., Hwu, W. J., Topalian, S. L., Hwu, P., Drake, C. G., Camacho, L. H., Kauh, J., Odunsi, K., et al. (2012). Safety and activity of anti-PD-L1 antibody in patients with advanced cancer. N Engl J Med 366, 2455-2465.
Carbone, D. P., Reck, M., Paz-Ares, L., Creelan, B., Horn, L., Steins, M., Felip, E., van den Heuvel, M. M., Ciuleanu, T. E., Badin, F., et al. (2017). First-Line Nivolumab in Stage IV or Recurrent Non-Small-Cell Lung Cancer. N Engl J Med 376, 2415-2426.
Carpentier, A., Metellus, P., Ursu, R., Zohar, S., Lafitte, F., Barrié, M., Meng, Y., Richard, M., Parizot, C., Laigle-Donadey, F., et al. (2010). Intracerebral administration of CpG oligonucleotide for patients with recurrent glioblastoma: a phase II study. Neuro Oncol 12, 401-408.
Ettinger, D. S., Wood, D. E., Akerley, W., Bazhenova, L. A., Borghaei, H., Camidge, D. R., Cheney, R. T., Chirieac, L. R., D'Amico, T. A., Dilling, T. J., et al. (2016). NCCN Guidelines Insights: Non-Small Cell Lung Cancer, Version 4.2016. J Natl Compr Canc Netw 14, 255-264.
Gosu, V., Basith, S., Kwon, O. P., and Choi, S. (2012). Therapeutic applications of nucleic acids and their analogues in Toll-like receptor signaling. Molecules 17, 13503-13529.
Govindan, R., Szczesna, A., Ahn, M. J., Schneider, C. P., Gonzalez Mella, P. F., Barlesi, F., Han, B., Ganea, D. E., Von Pawel, J., Vladimirov, V., et al. (2017). Phase III Trial of Ipilimumab Combined With Paclitaxel and Carboplatin in Advanced Squamous Non-Small-Cell Lung Cancer. J Clin Oncol 35, 3449-3457.
Guerra, C., Mijimolle, N., Dhawahir, A., Dubus, P., Barradas, M., Serrano, M., Campuzano, V., and Barbacid, M. (2003). Tumor induction by an endogenous K-ras oncogene is highly dependent on cellular context. Cancer Cell 4, 111-120.
Hameyer, D., Loonstra, A., Eshkind, L., Schmitt, S., Antunes, C., Groen, A., Bindels, E., Jonkers, J., Krimpenfort, P., Meuwissen, R., et al. (2007). Toxicity of ligand-dependent Cre recombinases and generation of a conditional Cre deleter mouse allowing mosaic recombination in peripheral tissues. Physiol Genomics 31, 32-41.
Hellmann, M. D., Rizvi, N. A., Goldman, J. W., Gettinger, S. N., Borghaei, H., Brahmer, J. R., Ready, N. E., Gerber, D. E., Chow, L. Q., Juergens, R. A., et al. (2017). Nivolumab plus ipilimumab as first-line treatment for advanced non-small-cell lung cancer (CheckMate 012): results of an open-label, phase 1, multicohort study. Lancet Oncol 18, 31-41.
Hemmi, H., Takeuchi, O., Kawai, T., Kaisho, T., Sato, S., Sanjo, H., Matsumoto, M., Hoshino, K., Wagner, H., Takeda, K., and Akira, S. (2000). A Toll-like receptor recognizes bacterial DNA. Nature 408, 740-745.
Herbst, R. S., Soria, J. C., Kowanetz, M., Fine, G. D., Hamid, O., Gordon, M. S., Sosman, J. A., McDermott, D. F., Powderly, J. D., Gettinger, S. N., et al. (2014). Predictive correlates of response to the anti-PD-L1 antibody MPDL3280A in cancer patients. Nature 515, 563-567.
Hornung, V., Rothenfusser, S., Britsch, S., Krug, A., Jahrsdörfer, B., Giese, T., Endres, S., and Hartmann, G. (2002). Quantitative expression of toll-like receptor 1-10 mRNA in cellular subsets of human peripheral blood mononuclear cells and sensitivity to CpG oligodeoxynucleotides. J Immunol 168, 4531-4537.
Jonkers, J., Meuwissen, R., van der Gulden, H., Peterse, H., van der Valk, M., and Berns, A. (2001). Synergistic tumor suppressor activity of BRCA2 and p53 in a conditional mouse model for breast cancer. Nat Genet 29, 418-425.
Karbach, J., Neumann, A., Atmaca, A., Wahle, C., Brand, K., von Boehmer, L., Knuth, A., Bender, A., Ritter, G., Old, L. J., and Jager, E. (2011). Efficient in vivo priming by vaccination with recombinant NY-ESO-1 protein and CpG in antigen naive prostate cancer patients. Clin Cancer Res 17, 861-870.
Li, K., Qu, S., Chen, X., Wu, Q., and Shi, M. (2017). Promising Targets for Cancer Immunotherapy: TLRs, RLRs, and STING-Mediated Innate Immune Pathways. Int J Mol Sci 18.
Medzhitov, R. (2001). Toll-like receptors and innate immunity. Nat Rev Immunol 1, 135-145.
Mikulandra, M., Pavelic, J., and Glavan, T. M. (2017). Recent Findings on the Application of Toll-like Receptors Agonists in Cancer Therapy. Curr Med Chem 24, 2011-2032.
Novello, S., Barlesi, F., Califano, R., Cufer, T., Ekman, S., Levra, M. G., Kerr, K., Popat, S., Reck, M., Senan, S., et al. (2016). Metastatic non-small-cell lung cancer: ESMO Clinical Practice Guidelines for diagnosis, treatment and follow-up. Ann Oncol 27, v1-v27.
Onizuka, S., Tawara, I., Shimizu, J., Sakaguchi, S., Fujita, T., and Nakayama, E. (1999). Tumor rejection by in vivo administration of anti-CD25 (interleukin-2 receptor alpha) monoclonal antibody. Cancer Res 59, 3128-3133.
Rawlins, E. L., Okubo, T., Xue, Y., Brass, D. M., Auten, R. L., Hasegawa, H., Wang, F., and Hogan, B. L. (2009). The role of Scgb1a1+ Clara cells in the long-term maintenance and repair of lung airway, but not alveolar, epithelium. Cell Stem Cell 4, 525-534.
Scheiermann, J., and Klinman, D. M. (2014). Clinical evaluation of CpG oligonucleotides as adjuvants for vaccines targeting infectious diseases and cancer. Vaccine 32, 6377-6389.
Schneberger, D., Caldwell, S., Kanthan, R., and Singh, B. (2013). Expression of Toll-like receptor 9 in mouse and human lungs. J Anat 222, 495-503.
Shi, M., Chen, X., Ye, K., Yao, Y., and Li, Y. (2016). Application potential of toll-like receptors in cancer immunotherapy: Systematic review. Medicine (Baltimore) 95, e3951.
Smith, J. B., and Wickstrom, E. (1998). Antisense c-myc and immunostimulatory oligonucleotide inhibition of tumorigenesis in a murine B-cell lymphoma transplant model. J Natl Cancer Inst 90, 1146-1154.
Tanaka, A., and Sakaguchi, S. (2017). Regulatory T cells in cancer immunotherapy. Cell Res 27, 109-118.
Topalian, S. L., Hodi, F. S., Brahmer, J. R., Gettinger, S. N., Smith, D. C., McDermott, D. F., Powderly, J. D., Carvajal, R. D., Sosman, J. A., Atkins, M. B., et al. (2012). Safety, activity, and immune correlates of anti-PD-1 antibody in cancer. N Engl J Med 366, 2443-2454.
Ursu, R., Carpentier, A., Metellus, P., Lubrano, V., Laigle-Donadey, F., Capelle, L., Guyotat, J., Langlois, O., Bauchet, L., Desseaux, K., et al. (2017). Intracerebral injection of CpG oligonucleotide for patients with de novo glioblastoma-A phase II multicentric, randomised study. Eur J Cancer 73, 30-37.
Yang, Y., Huang, C. T., Huang, X., and Pardoll, D. M. (2004). Persistent Toll-like receptor signals are required for reversal of regulatory T cell-mediated CD8 tolerance. Nat Immunol 5, 508-515.

## Claims

1. A pharmaceutical combination, comprising the components:
a) at least one regulatory T cell (Treg)-depleting agent,
b) at least one Toll-like receptor 9 (TLR9) agonist,
c) one or more immune checkpoint inhibitors,
for use in the treatment of cancer in humans or non-human mammals.

2. The combination according to claim 1, wherein the Treg-depleting agent of component a) is a surface antigen-depleting antibody or antibody fragment.

3. The combination according to claim 2, wherein the Treg-depleting agent of component a) is an antibody or antibody fragment directed against CD25, CD15s, GITR, CCR4, CTLA-4, OX-40, LAG3, GARP, ZAP-70 or PD-1 surface antigen.

4. The combination according to claim 1, wherein the Treg-depleting agent of component a) is phosphatidylinositol-4,5-bisphosphate 3-kinase delta (PI3Kδ) inhibitor.

5. The combination according to anyone claims 1 to 4, wherein the TLR9 agonist of component b) is composed of CpG oligodeoxynucleotides (CpG ODN) containing unmethylated CpG dinucleotides.

6. The combination according to claim 5, wherein the CpG ODN is composed of the formula CCx(not-C)(not-C)xxGGG, wherein x is any base selected from the group consisting of modified or unmodified A, T, C, G or derivatives thereof.

7. The combination according to claim 5 or claim 6, wherein the CpG ODN contains one or more nuclease-resistant phosphorothioate oligodeoxynucleotides.

8. The combination according to anyone of claim 5, wherein the CpG ODN comprises one or more of the nucleic acid sequences:
5'-TCGTCGTTTTGTCGTTTTGTCGTT-3' (SEQ ID NO: 1),
5'-GGGGGACGATCGTCGGGGGG -3' (SEQ ID NO: 2),
5'-TCGTCGTTTTCGGCGCGCGCCG-3' (SEQ ID NO: 3),
5'-GGGGTCAACGTTGAGGGGGG -3' (SEQ ID NO: 4),
5'-TCCATGACGTTCCTGACGTT-3' (SEQ ID NO: 5),
5'-TCCATGACGTTCCTGATGCT-3' (SEQ ID NO: 6),
5'-TCGACGTTCGTCGTTCGTCGTTC-3' (SEQ ID NO: 7),
5'-TCGTCGTTGTCGTTTTGTCGTT-3' (SEQ ID NO: 8),
5'-TCGCGACGTTCGCCCGACGTTCGGTA-3 (SEQ ID NO: 9),
5'-GGGGACGACGTCGTGGGGGGG-3' (SEQ ID NO: 10),
5'-TCGTCGTCGTTCGAACGACGTTGAT-3' (SEQ ID NO: 11),
5'-TCGCGAACGTTCGCCGCGTTCGAACGCGG-3' (SEQ ID NO: 12).

9. The combination according to anyone claims 1 to 8, wherein the TLR9 agonist of component b) is single-stranded or double-stranded genomic DNA from *Escherichia coli* or other prokaryotes and viruses with the ability to bind to TLR9 or those that mimic unmethylated CpG sequences in bacterial or viral DNA TLR9 agonists such as phosphodiester backbone and fold in a dumbbell-like structures known as double stem-loop immunomodulators (dSLIMs) with the ability to activate TLR9.

10. The combination according to anyone of claims 1 to 9, wherein the one or more checkpoint inhibitors inhibits a checkpoint protein selected from the group consisting of CTLA4, PD-1, PD-L1, PD-L2, LAG3, B7-H3, B7-H4, KIR, OX40, IgG, IDO-1, IDO-2, CEACAM1, TNFRSF4, OX40L, TIM3, BTLA, HVEM, TIM3, GAL9, LAG3, VISTA, KIR, 2B4, CD 160, CGEN-15049, CHK 1, CHK2, A2aR, and B-7or combinations thereof.

11. The combination according to claim 10, wherein the immune checkpoint inhibitor is a combination of αCTLA-4 and αPD-1 antibodies diluted in a single injection solution.

12. The combination according to anyone of claims 1 to 7, wherein the combination further comprises a dosage regime which provides that component a) is to be administered once or repeatedly before component b) and component c), and wherein optionally component b) is to be administered once or repeatedly before administering component c) to a cancer patient.

13. The combination according to anyone of claim 12, wherein the dosage regimen provides that
a. component a) is to be injected 5 to 9 days prior to day +1,
b. component b) is to be injected every 3^{rd} or 4^{th} day starting at day +1 by one or more injections,
c. component c) is to be injected every 3^{rd} or 4^{th} day starting at day +1 by one or more injections.

14. The combination according to anyone of claims 1 to 13, wherein all three components a) to c) are enclosed in separate injection solutions for subsequent injections to a cancer patient.

15. The combination according to anyone of claims 1 to 14, wherein the combination is a kit of parts.

16. An ex-vivo method for diminishing growth of tumour cells in a tissue, comprising the steps of:
a. application of at least one regulatory T cell (Treg)-depleting agent,
b. application of at least one Toll-like receptor 9 (TLR9) agonist,
c. application of one or more immune checkpoint inhibitors, wherein the at least TLR9 agonist is subsequently applied after Treg cell depletion, and wherein the one or more immune check point inhibitors are applied subsequently to or simultaneously with the at least one TLR9 agonist.

17. The ex-vivo method according to claim 16, wherein the Treg-depleting agent, the TLR9 agonist and/or the checkpoint inhibitors are any ones as defined in claims 1 to 14.
